# EUROPÄISCHE PATENTANMELDUNG

(11) **EP 1 356 776 A1**
(43) Veröffentlichungstag der Anmeldung: **29.10.2003**
(21) Anmeldenummer: 03005284.9
(22) Anmeldetag: 11.03.2003
(51) Int. Cl.: A61B 17/16, A61B 17/32

(54) **Codiersystem zur Kennzeichnung rotierender Werkzeuge in medizinischen Geräten**

(30) Priorität: 22.04.2002 DE 10217811
(71) Anmelder: Richard Wolf GmbH, 75438 Knittlingen (DE)
(72) Erfinder: Hill, Uwe, Dipl.-Ing. (FH), 75203 Königsbach-Stein (DE); Körner, Eberhard Dipl.-Ing. (FH), 75015 Bretten (DE); König, Steffen Dipl.-Ing. (FH), 76684 Östringen (DE)
(74) Vertreter: Hemmer, Arnd, Dipl.-Ing.

(57) **Zusammenfassung**

Die Erfindung betrifft ein Codiersystem zur Kennzeichnung von Werkzeugen in medizinischen Geräten sowie ein entsprechendes Verfahren, bei welchem an einer Außenseite eines Werkzeuges (2) aufeinanderfolgende Codeelemente (16; 20) angebracht sind und an einem feststehenden Teil eines Gerätes in der Nähe des Werkzeuges eine feststehende Leseeinrichtung (14) angeordnet ist zum Erfassen der aufeinanderfolgenden Codeelemente (16; 20) bei Bewegung des Werkzeuges (2) relativ zu dem feststehenden Teil.

## Beschreibung

Die Erfindung betrifft ein Codiersystem zur Kennzeichnung rotierender Werkzeuge in medizinischen Geräten sowie ein entsprechendes Verfahren zur Kennzeichnung rotierender Werkzeuge in medizinischen Geräten.

Medizinische Geräte mit rotierenden Werkzeugen werden z.B. als Gewebe-, Knorpel- und/oder Knochenfräsen eingesetzt, wie sie beispielsweise in der Arthroskopie zur Durchführung operativer Eingriffe in Körpergelenken Verwendung finden. Diese Instrumente bestehen im Wesentlichen aus einem in die Gelenkhöhle einzuführenden, rotierend angetriebenen Werkzeug und einem das Werkzeug antreibenden Motorhandgriff. Im Regelfall steht zur Durchführung der chirurgischen Eingriffe eine Vielzahl von Werkzeugeinsätzen zur Verfügung, welche wahlweise mit dem Motorhandgriff verbunden werden können. Die unterschiedlichen Werkzeuge erfordern jedoch häufig unterschiedliche Betriebsparameter, wie beispielsweise Werkzeugdrehzahl, so dass eine entsprechende Einstellung bzw. Ansteuerung des Motors erforderlich ist.

Zur Vornahme dieser Einstellung sind bereits Systeme bekannt, bei welchen an dem Werkzeug eine Codierung vorgesehen ist, welche von einem entsprechenden Sensor an dem Motorhandgriff erfasst wird, um beispielsweise die Drehzahl des Motors abhängig von dem verwendeten Werkzeug automatisch einzustellen. Hierzu ist es beispielsweise aus US 4,705,038, US 5,217,478 und WO 97/16124 bekannt, magnetische Codierungen an den Werkzeugen vorzusehen, welche von einem entsprechenden Sensor in dem Handgerät bzw. Motorhandgriff erfasst werden. Diese Codierungen sind so ausgebildet, dass Magneten, die von Reed- oder Hallsensoren in dem Handgerät erfasst werden, in einer feststehenden Hülse angeordnet sind, welche die rotierenden Werkzeugteile umgibt. Diese feststehende Hülse wird gemeinsam mit dem Werkzeug von dem Handteil zum Austausch des Werkzeuges gelöst. Die feststehende Hülse bildet mit dem Werkzeug eine Einheit, wobei das Werkzeug in der Hülse drehbar ist. Wird das Werkzeug mit dem Handteil verbunden, so wird die Hülse drehfest an dem Handteil gehalten, während das Werkzeug, angetrieben durch den Motor, rotieren kann. Auf diese Weise können in dem Handteil an bestimmten Positionen angeordnete Sensoren, wie Reed- oder Hallsensoren, erfassen, ob an korrespondierenden, vorgegebenen Stellen in der Hülse Magnete angeordnet sind oder nicht. Je nachdem, wie viele mögliche Magnetpositionen und entsprechend angeordnete Sensoren vorgesehen sind, kann ein begrenzte Anzahl von unterschiedlichen Codes an dem Werkzeug zu dessen Identifizierung eingestellt werden.

Diese Anordnung hat jedoch den Nachteil, dass zur Codierung einer großen Anzahl unterschiedlicher Werkzeuge eine große Anzahl von Sensoren an verschiedenen Positionen des Handteils erforderlich ist. Ferner ist der Informationsumfang, der in einem solchen Code abgelegt werden kann, begrenzt, so dass es über die reine Werkzeugidentifizierung hinaus schwierig ist, weitere Informationen in der an dem Werkzeug vorgesehenen Codierung abzulegen. Beispielsweise kann es wünschenswert sein, neben einer reinen Identifizierung eines Werkzeuges direkt die erforderliche Motordrehzahl in codierter Form in dem Werkzeugcode abzulegen. Ferner ist es denkbar, weitere Informationen wie den erforderlichen Spülflüssigkeitsfluss etc. in dieser Codierung abzulegen.

Es ist daher Aufgabe der Erfindung, ein Codiersystem sowie ein Verfahren zur Kennzeichnung von Werkzeugen in medizinischen Geräten zu schaffen, welche eine vereinfachte Codierung mit einem größeren Informationsumfang ermöglichen.

Diese Aufgabe wird durch ein Codiersystem mit den im Anspruch 1 angegebenen Merkmalen sowie durch ein Verfahren mit den im Anspruch 11 angegebenen Merkmalen gelöst. Bevorzugte Ausführungsformen ergeben sich aus den Unteransprüchen.

Bei dem erfindungsgemäßen Codiersystem zur Kennzeichnung von Werkzeugen in medizinischen Geräten wird die Codierung an dem Werkzeug selber angebracht. Das bedeutet, die Codierung bewegt sich mit dem Werkzeug relativ zu einem feststehenden Teil des medizinischen Geräts, welcher üblicherweise ein Handgerät bzw. Motorhandgriff ist.

An einem rotierenden Werkzeug sind vorzugsweise an einer Außenseite in Umfangsrichtung aufeinanderfolgende Codeelemente angebracht, und an einem feststehenden Teil des Geräts ist in der Nähe des rotierenden Werkzeugs eine feststehende Leseeinrichtung angeordnet. Auf diese Weise rotieren bei Rotation des Werkzeuges die Codeelemente relativ zu dem feststehenden Teil des Gerätes bzw. zu der feststehenden Leseeinrichtung, welche die Codeelemente erfasst. Dabei bewegen sich die einzelnen Codeelemente nacheinander an der feststehenden Leseeinrichtung vorbei und können dabei von dieser ausgelesen werden. Dabei wird der durch die Codeelemente dargestellte Code seriell beim Passieren der Leseeinrichtung ausgelesen. Es wird somit die ohnehin mögliche bzw. stattfindende Rotation des Werkzeuges ausgenutzt, um die einzelnen Codeelemente nacheinander an der Leseeinrichtung vorbeizubewegen. Es ist somit möglich, einen sehr komplexen Code mit großem Informationsumfang mit nur einem Sensorelement in Form der Leseeinrichtung auszulesen. Dies ermöglicht, eine wesentlich größere Anzahl von Werkzeugen entsprechend zu codieren. Ferner kann ein größerer Informationsgehalt der Codierung erreicht werden, was ermöglicht, auch weitere Betriebsparameter in dem Code an dem Werkzeug abzulegen, beispielsweise die erforderliche Drehzahl, die erforderliche Menge von Spülflüssigkeit etc. Diese Daten können dann automatisch durch die Leseeinrichtung ausgelesen und zur Steuerung des Motors bzw. Antriebs in dem feststehenden Teil des Geräts verwendet werden. Der Informationsumfang, der in dem Code abgelegt werden kann, hängt vor allem von der Auflösung des Lesegerätes ab. Bei einem hohen Auflösevermögen des Lesegerätes können die Codeelemente entsprechend klein ausgebildet werden, so dass eine größere Anzahl von Codeelementen auf einem vorgegebenen Streckenabschnitt, beispielsweise dem Umfang des Werkzeuges, angeordnet werden kann.

Alternativ oder zusätzlich zu der zuvor beschriebenen Ausführungform können die Codeelemente in Längsrichtung des Werkzeuges aufeinander folgend angeordnet sein und bei einer Bewegung des Werkzeuges in seiner Längsrichtung relativ zu dem feststehenden Teil von der Leseeinrichtung erfassbar sein. Bei dieser Anordnung werden die Codeelemente ebenfalls durch eine relative Bewegung des Werkzeuges zu einer am feststehenden Teil des Gerätes ausgebildeten Leseeinrichtung ausgelesen. Allerdings erfolgt das Auslesen im Unterschied zu dem zuvor genannten Ausführungsbeispiel nicht durch Rotation des Werkzeuges, sondern durch Linearbewegung in Längsrichtung des Werkzeuges. So können die Codeelemente beispielsweise beim Einsetzen des Werkzeuges in einen feststehenden Teil des Gerätes ausgelesen werden. Beispielsweise wird das Werkzeug in seiner Längsrichtung in eine entsprechende Aufnahme an dem feststehenden Teil des Gerätes eingesteckt. Dabei erfolgt eine Linearbewegung in Längsrichtung des Werkzeuges, durch welche die Codeelemente an der feststehenden Leseeinrichtung vorbeibewegt werden, um von der Leseeinrichtung ausgelesen werden zu können. Auch bei dieser Ausführungsform wird vorzugsweise eine ohnehin beim Betrieb bzw. Einsatz des Werkzeuges erforderliche Relativbewegung zwischen Werkzeug und feststehendem Geräteteil ausgenutzt, um die Codeelemente an einer Leseeinrichtung vorbeizuführen. Die Anordnung, bei welcher die Codeelemente in Längsrichtung des Werkzeuges aufeinander folgend angeordnet sind, eignet sich nicht nur für rotierende Werkzeuge, sondern auch für feststehende Werkzeuge, welche austauschbar an einem feststehenden Geräteteil angeordnet werden sollen, beispielsweise an einem Griffteil. Ferner ist auch eine Kombination von in Umfangsrichtung und in Längsrichtung angeordneten Codeelementen möglich, um komplexere Codes mit größerem Informationsgehalt zu ermöglichen.

Vorzugsweise weisen die Codeelemente eine Vielzahl zueinander paralleler, streifenförmiger Elemente auf, welche in Umfangsrichtung und/oder Längsrichtung des Werkzeuges in unterschiedlichen Abständen zueinander und/oder unterschiedlichen Breiten an der Oberfläche des Werkzeuges zu dessen Kennzeichnung ausgebildet sind. Die Codeelemente bilden somit einen Strichcode, wie er grundsätzlich bekannt ist. Zum Auslesen werden die einzelnen, streifenförmigen Elemente nacheinander durch die Rotation oder Axialbewegung des Werkzeuges an der feststehenden Leseeinrichtung vorbeibewegt und von dieser erfasst bzw. ausgelesen. Je nachdem, wie fein die Auflösung des Streifen- bzw. Strichrasters ist, in dem die streifenförmigen Elemente angeordnet sind, kann eine bestimmte Anzahl unterschiedlicher Codes realisiert werden. Da derartige Strichcodes sehr fein ausgebildet werden können, ist es möglich, dass eine große Anzahl verschiedener Werkzeuge entsprechend codiert werden kann. Die streifenförmigen Elemente sind vorzugsweise parallel zur Drehachse des Werkzeuges an dessen Umfang angeordnet, so dass sie sich bei Rotation in Umfangsrichtung, d. h. quer zu ihrer Erstreckungsrichtung, an der Leseeinrichtung vorbeibewegen.

In einer weiteren bevorzugten Ausführungsform weisen die Codeelemente eine Vielzahl von streifenförmigen oder kreissegmentförmigen Elementen auf, welche mit unterschiedlichen Abständen zueinander und/oder unterschiedlichen Breiten ausgebildet sind und entlang einer Kreisebene in einer Ebene quer zur Drehachse des Werkzeuges an dem Werkzeug angeordnet sind. Die Art der Codierung und des Auslesens erfolgt entsprechend zu der oben beschriebenen Ausführungsform. Allerdings erstrecken sich bei dieser zweiten Ausführungsform die streifenoder kreissegmentförmigen Elemente im Wesentlichen radial zur Drehachse des Werkzeuges. Die Codeelemente sind somit ringförmig in einer Ebene quer zur Rotationsachse des Werkzeuges angeordnet, beispielsweise an einer Stirnseite des Werkzeuges. Dabei wird die Leseeinrichtung so angeordnet, dass sich auch hier die streifenförmigen oder kreissegmentförmigen Elemente in Umfangsrichtung des Werkzeuges, d.h. im Wesentlichen quer zur Erstreckungsrichtung der streifenförmigen oder kreissegmentförmigen Elemente, relativ an der Leseeinrichtung vorbeibewegen, wenn das Werkzeug gedreht wird.

Weiter bevorzugt sind die Codeelemente als Stege ausgebildet. Hierzu können Nuten oder Kerben an dem Werkzeug ausgebildet werden, zwischen denen die Codeelemente in Form von Stegen entstehen. Entsprechend ist es auch möglich, Vorsprünge an dem Werkzeug vorzusehen. Die Leseeinrichtung ist dabei entsprechend beschaffen, so dass sie erkennen kann, ob an einer bestimmten Position Material vorhanden ist oder nicht, d. h. die Leseeinrichtung erfasst die Stege oder die Ausnehmungen zwischen den Stegen.

Die Codeelemente können an einer Hülse oder Scheibe ausgebildet sein, welche mit dem Werkzeug drehfest verbunden ist. Dies ermöglicht, die Codeelemente unabhängig von dem Werkzeug herzustellen und in der Fertigung nachträglich mit dem Werkzeug zu verbinden. Beispielsweise können die Codeelemente als Stege in einem hülsen- oder scheibenförmigen Blech ausgebildet sein. Dies kann beispielsweise durch Ausstanzen erfolgen, wobei die Stege zwischen den ausgestanzten Ausnehmungen stehen bleiben.

Vorzugsweise weist das Werkzeug einen feststehenden Gehäuseteil und einen darin angeordneten beweglichen Teil auf, wobei die Codeelemente im Inneren des Gehäuseteils angeordnet sind. Der feststehende Teil wird beispielsweise von einem Schaft und einem sich anschließenden Kupplungsgehäuse gebildet, in deren Inneren eine drehbare oder längsverschiebbare Welle bzw. eine Kupplung angeordnet sind. Der feststehende Gehäuseteil, d. h. das Kupplungsgehäuse wird mit dem feststehenden Teil des Gerätes verbunden, während die Kupplung und damit die Welle mit einer Antriebseinrichtung im Inneren des Gerätes zur Bewegung der Welle verbunden wird. Bevorzugt sind die Codeelemente, welche an dem beweglichen Teil des Werkzeuges angeordnet sind, so angeordnet, dass sie sich im Inneren des feststehenden Gehäuseteils befinden. Bevorzugt werden die Codeelemente im Bereich der Kupplung im Inneren eines Kupplungsgehäuses angeordnet. Dadurch sind die Codeelemente durch den feststehenden Gehäuseteil nach außen vor Verschmutzungen und Beschädigungen geschützt. Dies ist bei medizinischen Geräten wichtig, da sich diese leicht reinigen lassen müssen und möglichst vor Verunreinigungen an schwer zu reinigenden Stellen geschützt werden müssen. Insbesondere wenn die Codeelemente in Form von Ausnehmungen oder Vorsprüngen ausgebildet sind, sind diese im Inneren des feststehenden Gehäuseteils vor Verunreinigungen geschützt. Eine feststehende Leseeinrichtung ist an oder in dem feststehenden Teil des Gerätes angeordnet. Dabei kann die Leseeinrichtung außerhalb des feststehenden Gehäuseteils des Werkzeuges angeordnet sein und die Codeelemente durch das Gehäuseteil bzw. eine Wandung des Gehäuseteils hindurch erfassen. Beispielsweise kann das Gehäuseteil in Form eines Kupplungsgehäuses oder Schaftes aus Kunststoff ausgebildet sein und ein magnetfeldbeeinflussendes Codeelement durch eine solche Kunststoffwandung hindurch erfasst werden. Auch eine optische Erfassung ist bei transparenter Ausbildung der Gehäusewandung möglich. Alternativ kann die feststehende Leseinrichtung in den feststehenden Gehäuseteil des Werkzeuges hineinragen bzw. eingreifen.
Weiter bevorzugt sind die Codeelemente im Inneren des beweglichen Teils des Werkzeuges angeordnet. Beispielsweise können die Codeelemente in einer Kupplung angeordnet sein, welche am proximalen Ende des beweglichen Teils des Werkzeuges zur Verbindung mit einer Antriebseinrichtung vorgesehen ist. Dabei sind die Codeelemente bevorzugt im Inneren einer hohl bzw. hülsenförmig ausgebildeten Kupplung angeordnet. Die Codeelemente können auch in das Material der Kupplung integriert sein, beispielsweise mit dieser vergossen sein. So können Codeelemente aus Metall in eine Kupplung aus Kunststoff eingegossen sein. Die Erfassung der Codeelemente erfolgt, wie vorangehend beschrieben, auch bei dieser Ausführungsform durch eine am oder im feststehenden Geräteteil angeordnete Leseeinrichtung, welche die Codeelemente z.B. optisch oder magnetisch erfassen kann.

Dabei sind die Codeelemente vorzugsweise magnetfeldbeeinflussend ausgebildet, und die Leseeinrichtung erfasst Änderungen eines Magnetfeldes. Die Leseeinrichtung kann beispielsweise ein Hallsensor sein, welcher Änderungen des Magnetfeldes erfasst. Diese Änderungen des Magnetfeldes können dadurch erfolgen, dass die Codeelemente in Form von Kerben oder Stegen bzw. Vorsprüngen ausgebildet sind, welche beim Passieren der Leseeinrichtung eine Änderung des Magnetfeldes bewirken, welche von der Leseeinrichtung erfasst wird. Die Leseeinrichtung ist somit als Zahnradsensor ausgebildet. Ein solcher Zahnradsensor kann beispielsweise in bekannter Weise aus einem Hallsensor bestehen, der auf einem Permanentmagneten aufgebracht ist. Über den Sensor werden dann die durch die Codeelemente verursachten Veränderungen des Magnetfeldes erfasst. Alternativ ist es auch denkbar, Codeelemente aus magnetischem Material an dem Werkzeug anzubringen, welche durch die Leseeinrichtung erfasst werden. Dazu kann die Leseeinrichtung beispielsweise auch als Reedkontakt ausgebildet sein.

Alternativ können die Codeelemente und die Leseeinrichtung derart ausgebildet sein, dass die Codeelemente durch die Leseeinrichtung optisch erfassbar sind. Hierzu können beispielsweise an dem Werkzeug Codeelemente als reflektierende Elemente ausgebildet sein. Es kann ein Strichcode bestehend aus abwechselnd besser und schlechter reflektierenden Streifen, insbesondere reflektierenden und nicht reflektierenden Streifen unterschiedlicher Breite vorgesehen sein. Die Leseeinrichtung wirkt dann derart, dass die Codeelemente beleuchtet werden und Reflexion bzw. die Stärke der Reflexion von einem Sensor erfasst wird. In einfachster Ausgestaltung können dazu die Codeelemente als Barcode mit abwechselnd angeordneten schwarzen und weißen Streifen ausgebildet sein. Es ist jedoch auch möglich, die Codeelemente in einer Scheibe als Spalte oder Zähne auszubilden, welche bei Rotation einen Lichtstrahl unterbrechen oder freigeben, was von einem entsprechenden Sensor erfasst wird. Alle diese verschiedenen Ausgestaltungen von Codeelementen und Leseeinrichtungen haben gemeinsam, dass die Codeelemente gemeinsam mit dem Werkzeug rotieren und beim Passieren einer feststehenden Leseeinrichtung erfasst werden, so dass der von den Codeelementen gebildete Code seriell ausgelesen wird. Die Leseeinrichtung erzeugt dann ein entsprechendes Signal, welches an die Steuereinrichtung des Gerätes zum Antrieb des Motors weitergegeben wird.

Die Codeelemente können eine codierte Bezeichnung der Werkzeugart und/oder von Betriebsparametern für das Werkzeug darstellen. Bei der einfachsten Form der Codierung kennzeichnen die Codeelemente lediglich ein bestimmtes Werkzeug. Die Leseeinrichtung liest dann die Codierung aus und gibt diesen an eine Steuereinrichtung weiter, die an dem Code das Werkzeug erkennt und dann die Steuerung des Antriebs entsprechend dem eingesetzten Werkzeug vornimmt. Bei dieser Ausführungsform ist es erforderlich, dass die für bestimmte Werkzeuge erforderlichen Betriebsparameter in der Steuerung oder dem Gerät abgelegt sind und, nachdem das zugehörige Werkzeug erkannt ist, aufgerufen werden. Alternativ ist es möglich, dass der Code an dem Werkzeug selber die erforderlichen Betriebsparameter in codierter Form enthält. Diese werden dann von der Leseeinrichtung ausgelesen und an die Steuereinrichtung weitergegeben, welche dann das Gerät und insbesondere den Antrieb des Werkzeuges entsprechend steuert. Diese zweite Ausführungsform hat den Vorteil, dass leicht neue Werkzeuge eingesetzt werden können, ohne dass deren Betriebsparameter vorher der Steuereinrichtung eingegeben werden müssen. Die Steuereinrichtung erfasst dabei nicht nur einen bestimmten Werkzeugtyp, sondern gleich Informationen darüber, welche Betriebsparameter eingestellt werden müssen.

Die Codeelemente können zusätzlich Positionsmarkierungen zur Bestimmung der Winkelposition und/oder Drehzahl des Werkzeuges beinhalten. Hierzu können spezielle Codeelemente vorgesehen sein. Alternativ ist es insbesondere zur Drehzahlerfassung möglich, auf zusätzliche Codeelemente zu verzichten, vielmehr wird lediglich die Wiederholung eines beliebigen, an dem Werkzeug vorgesehenen Codemusters erfasst und zur Drehzahlbestimmung verwendet. Insgesamt hat diese Ausgestaltung den Vorteil, dass auf zusätzliche Positions- und Drehzahlsensoren verzichtet werden kann, da diese Funktion von dem Codiersystem mit übernommen werden kann. Es kann auch eine bestimme Anfangsmarkierung vorgesehen sein, um den Anfang eines auszulesenden Codes zu markieren, um der Leseeinrichtung bei Rotation des Werkzeuges eine Information darüber zu geben, an welcher Stelle des Werkzeugumfanges oder des ringförmig angeordneten Codes der Anfang der Codierung liegt. Die Anfangsmarkierung kann gegebenenfalls gleichzeitig zur Positions- und Drehzahlbestimmung verwendet werden. Die Codeelemente können auch zur Deaktivierung der Antriebseinrichtung verwendet werden, beispielsweise wenn kein Werkzeug in den Handgriff eingesetzt ist.

Bevorzugt stellen die Codeelemente digitale Informationen dar. Dies geschieht dadurch, dass die Codeelemente in einem vorgegebenen Raster angeordnet werden. Beispielsweise kann eine Streifeneinteilung entlang dem Werkzeugumfang vorgesehen sein. Jeder Streifen kann zwei Informationszustände haben, beispielsweise reflektierend oder nicht reflektierend sein. Alternativ kann der Streifen als Kerbe oder Steg bzw. Vorsprung bzw. als Kerbe oder Vollmaterial ausgebildet sein. Jeder Streifen kann somit zwei Zustände haben, welche von der Leseeinrichtung erfasst werden. Somit kann ein Binärcode in der Codierung dargestellt werden. Die Leseeinrichtung erfasst diese Codierung und gibt ein entsprechendes, digitales Signal aus, welches von einer Steuereinrichtung weiterverarbeitet werden kann.

Die Erfindung betrifft ferner ein entsprechendes Verfahren zur Kennzeichnung von Werkzeugen in medizinischen Geräten. Gemäß diesem Verfahren werden an einem bestimmten Werkzeug Codeelemente in einem vorbestimmten Muster zur Identifizierung des Werkzeuges oder Speicherung bestimmter Informationen angeordnet. Diese Informationen betreffen vorzugsweise Eigenschaften oder Betriebsparameter des Werkzeuges. Zum Auslesen der Informationen wird das Werkzeug relativ zu einem feststehenden Teil eines medizinischen Gerätes bewegt, wobei die Codeelemente eine feststehend ausgebildete Leseeinrichtung passieren. Die Leseeinrichtung erfasst bei der Bewegung des Werkzeuges die aufeinanderfolgenden Codeelemente und kann die durch die Codeelemente codiert abgelegte Information auslesen und an eine Steuereinrichtung weitergeben. Bei einer Vielzahl von Werkzeugen, welche mit einem medizinischen Gerät z. B. einem Handgerät kombiniert werden können, kann jedem Werkzeug ein individueller Code zugeordnet werden. Dabei ermöglicht das erfindungsgemäße Verfahren, eine große Anzahl unterschiedlicher Werkzeuge entsprechend zu codieren. Alternativ oder zusätzlich ist es möglich, direkt an jedem Werkzeug gemäß dem erfindungsgemäßen Codierverfahren codierte Informationen über einzustellende Betriebsparameter abzulegen. Diese können dann von der Leseeinrichtung ausgelesen und an eine Steuerung zur Steuerung des Gerätes weitergegeben werden.

Gemäß einer ersten bevorzugten Ausführungsform des Verfahrens werden die Codeelemente auf einer zur Drehachse des Werkzeuges konzentrischen Kreisbahn angeordnet und das Werkzeug wird zum Auslesen relativ zu dem feststehenden Teil eines Gerätes gedreht. Dabei kann eine relative Drehung des Werkzeuges ausgenutzt werden, welche ohnehin zum beabsichtigten Betrieb des Werkzeuges, beispielsweise bei einem Fräser oder Bohrer, erforderlich ist. Auf diese Weise wird eine ohnehin beim Betrieb des Werkzeuges erforderliche Bewegung ausgenutzt, um die erforderliche Relativbewegung zwischen Codeelementen und feststehender Leseeinrichtung zum Auslesen der Codeelemente zu erzeugen.

Alternativ oder zusätzlich zu dieser ersten Ausführungsform können die Codeelemente in Längsrichtung des Werkzeuges aufeinander folgend angeordnet werden und bei einer Linearbewegung des Werkzeuges in dessen Längsrichtung relativ zu dem feststehenden Teil des Gerätes von der Leseeinrichtung ausgelesen werden. Eine derartige Linearbewegung des Werkzeuges in seiner Längsrichtung erfolgt beispielsweise beim Einsetzten und Herausnehmen des Werkzeuges aus einem feststehenden Teil eines Gerätes, wie beispielsweise einem Handgriff. Auch gemäß dieser Ausführungsform wird eine ohnehin zum Betrieb des Werkzeuges erforderliche Bewegung des Werkzeuges ausgenutzt, um die zwischen Codeelementen und feststehender Leseeinrichtung notwendige Relativbewegung zu erzeugen. Es ist ferner möglich die lineare und umfängliche Anordnung von Codeelementen zu kombinieren, um komplexere Codes erzeugen zu können und verschiedene Codes bei unterschiedlichen Bewegungen des Werkzeuges auslesen zu können. Ferner eignet sich die lineare Anordnung der Codeelemente in Längsrichtung des Werkzeuges auch zur Codierung von nicht rotierenden Werkzeugen, welche austauschbar an feststehenden Geräteteilen angebracht werden können.

Nachfolgend wird die Erfindung beispielhaft anhand beigefügter Figuren beschrieben. In diesen zeigt:
- Fig. 1: eine schematische Schnittansicht eines Werkzeuges mit einer zugehörigen Leseeinrichtung,
- Fig. 2: eine Codierhülse gemäß einem ersten Ausführungsbeispiel,
- Fig. 3: eine Codierscheibe,
- Fig. 4: eine Schnittansicht einer Codierhülse gemäß Fig. 2 mit einem zugehörigen Signalverlauf und
- Fig. 5: eine Codierhülse gemäß einem zweiten Ausführungsbeispiel.

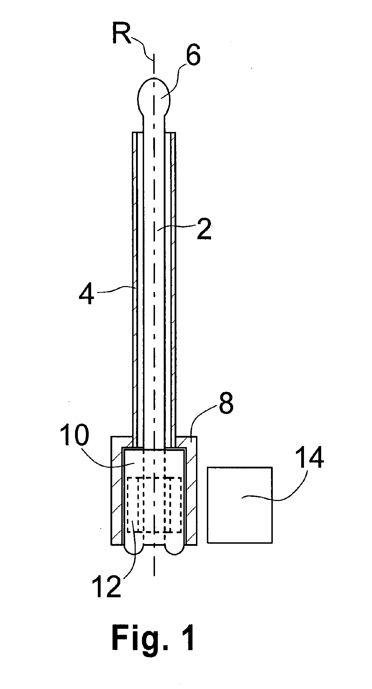

Fig. 1 zeigt eine schematische Schnittansicht eines Werkzeuges mit dem erfindungsgemäßen Codiersystem. Das Werkzeug besteht aus einer drehbaren Welle 2, welche in einem feststehenden Schaft 4 angeordnet oder geführt ist. An dem in Fig. 1 oberen Ende des Schafts 4 tritt die drehbare Welle 2 aus dem Schaft 4 aus und bildet das eigentliche Werkzeug bzw. den Werkzeugkopf 6, welcher die Bearbeitung durchführt. Der Schaft 4 ist an dem dem Werkzeugkopf 6 entgegengesetzten Ende mit einem Kupplungsteil 8 verbunden, welches in hier nicht gezeichneter Weise mit einem feststehenden Geräteteil wie einem Handgerät bzw. einem Motorhandgriff verbunden werden kann. Die drehbare Welle 2 weist ebenfalls an seinem dem Werkzeugkopf 6 entgegengesetzten Ende im Inneren des Kupplungsteils 8 eine Kupplung 10 zum Verbinden mit dem Antrieb in dem Handgerät auf. Die Kupplung 10 kann beispielsweise als Messerkupplung ausgebildet sein. Da diese Ausgestaltung bereits bekannt ist, wird hier auf eine detailliertere Beschreibung verzichtet. Im Bereich der Kupplung 10 ist drehfest verbunden mit der Welle 2 eine Codierhülse bzw. ein Target 12 angeordnet, welches die Welle 2 umfänglich umgibt. Die Codierhülse 12 weist in Umfangsrichtung verteilte Ausnehmungen mit dazwischen ausgebildeten Stegen von jeweils vorbestimmter Breite und vorbestimmtem Abstand zueinander auf, welche die Codeelemente bilden. Die in der Codierhülse 12 in Form von Ausnehmungen bzw. Stegen bestimmter Breite ausgebildeten Codeelemente beeinflussen ein Magnetfeld, welches von einer feststehenden Leseeinrichtung 14 erfasst wird. Die Leseeinrichtung 14 ist an dem feststehenden Geräteteil bzw. dem Handgerät nahe dem aufzunehmenden Werkzeug angeordnet.

Die Codierhülse 12 ist, wie in Fig. 1 gezeigt ist, im Inneren der Kupplung 10 angeordnet. Auf diese Weise wird die Codierhülse vor Verunreinigungen, welche sich in den Ausnehmungen bzw. Stegen festsetzen könnten, geschützt. Das Kupplungsgehäuse bzw. Kupplungsteil 8 wird, wenn das Werkzeug an ein Handgerät angesetzt wird, mit dem feststehenden Gehäuse des Handgerätes verbunden, so dass die Kupplung 10 und die in deren Inneren angeordnete Codierhülse 12 in einem abgeschlossenen Raum angeordnet und somit zusätzlich vor Verunreinigungen und Beschädigungen geschützt sind. Die Kupplung 10 tritt drehfest mit einer korrespondierenden Kupplung eines Antriebs in dem Handgerät in Eingriff. Die Leseeinrichtung 14 kann die an der Codierhülse ausgebildeten Codeelemente durch die Wandung des Kupplungsteils 8 und die Kupplung 10 hindurch erfassen. Um dies zu ermöglichen, ist die Wandung des Gehäuseteils 8 vorzugsweise aus Kunststoff ausgebildet, um eine störungsfreie Erfassung von Magnetfeldänderungen, welche bei Rotation durch die Codierhülse 12 im Inneren der Kupplung erzeugt werden, mittels der Leseeinrichtung 14 zu ermöglichen.

Wenn das Werkzeug mit dem Handgerät verbunden ist, wird die Welle 2 über die Kupplung 10 von einem Antrieb derart angetrieben, dass sie sich um die Rotationsachse R dreht. Dabei dreht sich auch die Codierhülse 12 entsprechend mit um die Rotationsachse R. Dabei passieren die einzelnen, in Umfangsrichtung hintereinander in der Codierhülse 12 angeordneten Codeelemente seriell die Leseeinrichtung 14 und werden von dieser erfasst. Die Leseeinrichtung 14 gibt ein den erfassten Codeelementen entsprechendes Signal aus, welches von einer Steuereinrichtung zur Steuerung des Antriebs des Schaftes 2 weiterverarbeitet wird.

Diese Anordnung hat den Vorteil, dass nur ein Sensorelement in Form der Leseeinrichtung 14 zum Erfassen einer Vielzahl von Codeelementen erforderlich ist. Auf diese Weise kann auf sehr einfache Art ein komplexer Code mit großem Informationsgehalt ausgelesen werden.

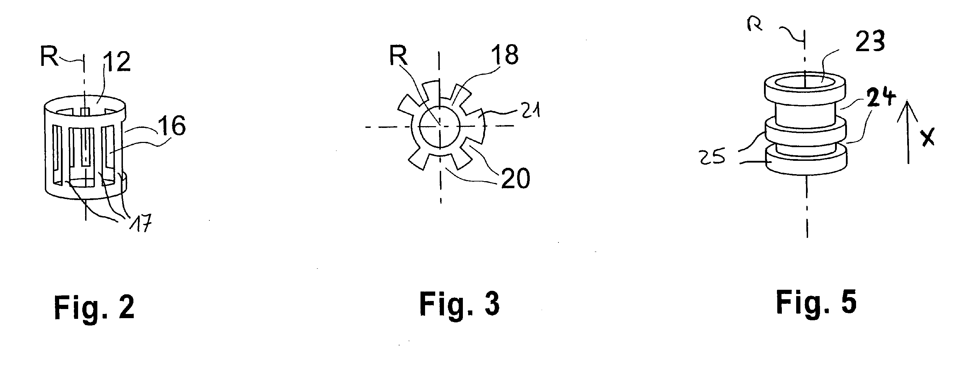

Fig. 2 zeigt eine perspektivische Detailansicht der Codierhülse 12. Die Codierhülse 12 ist ringförmig ausgebildet und wird konzentrisch zu der Rotationsachse R drehfest an dem Schaft 2 angeordnet. In der Codierhülse 12 sind in Umfangsrichtung aufeinanderfolgende Ausnehmungen 16 mit dazwischenliegenden Stegen 17 ausgebildet. Die Stege 17 bilden Codeelemente. Dabei können die einzelnen Stege 17 in Umfangsrichtung variierende Breiten und in Umfangsrichtung variierende Abstände zueinander aufweisen, um verschiedene Codierungen bzw. Codes in der Codierhülse 12 auszubilden. Der Code kann als binärer Code ausgebildet sein. Dazu ist der Umfang der Codierhülse 12 in streifenförmige Abschnitte aufgeteilt, wobei jeder streifenförmige Abschnitt zwei mögliche Zustände haben kann. Entweder ist in dem entsprechenden Abschnitt ein Steg oder eine Ausnehmung ausgebildet. Dabei können auch mehrere Ausnehmungen oder Stegbereiche direkt aufeinanderfolgen. Dies bewirkt, dass Ausnehmungen größerer Breite bzw. breitere Stege zwischen einzelnen Ausnehmungen entstehen. Die Codierhülse 12 bildet ein magnetfeldbeeinflussendes Target. Die Kupplung 10 ist vorzugsweise als Kunststoffkupplung ausgebildet, und die Leseeinrichtung 14 kann in bekannter Weise als Zahnradsensor ausgebildet sein. Wenn nun bei Rotation des Werkzeuges die einzelnen Ausnehmungen 16 bzw. Stege 17 nacheinander bzw. seriell die Leseeinrichtung 14 passieren, ändert sich seriell bzw. periodisch das Magnetfeld, was von der Leseeinrichtung erfasst wird. Auf diese Weise kann die Leseeinrichtung erkennen, ob an der entsprechenden Position bzw. dem entsprechenden Abschnitt der Codierhülse 12 eine Ausnehmung 16 oder ein Steg 17 vorhanden ist. Bei Rotation der Hülse 12 wird dabei durch die Leseeinrichtung 14 der gesamte Umfang der Hülse 12 seriell abgetastet. Eine bestimmte Ausgestaltung der Ausnehmung 16 bzw. des Steges 17 oder ein zusätzliches Codierelement kann als Positionsmarkierung dienen. Diese kann zum einen dazu dienen, die Anfangsposition des entlang dem Umfang der Hülse 12 ausgebildeten Codes zu bestimmen. Ferner können eine oder mehrere Positionsmarkierungen an dem Umfang dazu dienen, die Winkelposition der Welle oder deren Drehzahl zu bestimmen. Hierzu muss jedoch nicht eine zusätzliche Positionsmarkierung vorgesehen werden, vielmehr kann auch eine bestimmte Stelle des zur Werkzeugmarkierung bzw. Werkzeugidentifizierung dienenden Codes zur Positions- und Drehzahlbestimmung verwendet werden.

Fig. 3 zeigt eine alternative Ausführungsform der Erfindung. Hierbei sind die einzelnen Codeelemente nicht in einer ringförmigen Hülse, sondern am umfänglichen Rand einer kreisförmigen Scheibe 18 ausgebildet. Die Scheibe 18 wird derart drehfest mit dem Schaft 2 des Werkzeuges verbunden, dass sie sich quer bzw. normal zu der Rotationsachse R erstreckt. Am Umfang oder in Umfangsrichtung der Scheibe sind Ausnehmungen 20 mit dazwischenliegenden Stegen 21 ausgebildet, welche die Codeelemente bilden. Entsprechend der Ausgestaltung der Codierhülse 12 kann der Umfang der Codierscheibe 18 in vorbestimmte Segmente eingeteilt sein, welche jeweils zwei Zustände zur Codierung haben können. Entweder ist im Bereich eines bestimmten Segmentes eine Ausnehmung 20 oder ein Steg 21 vorgesehen. Folgen mehrere Segmente mit Ausnehmung aufeinander, entstehen Ausnehmungen 20 mit in Umfangsrichtung größerer Breite bzw. weiter voneinander beabstandete Stege 21. Entsprechend können auch breitere Stege 21 ausgebildet werden, wenn zwei Steg-Segmente aufeinanderfolgen. Die Scheibe 18 ist wie auch die Hülse 12 vorzugsweise aus einem ferromagnetischen Material ausgebildet, so dass die Ausnehmungen 20 bzw. Stege 21 wie auch die Ausnehmungen 16 bzw. Stege 17 beim Passieren der Leseeinrichtung 14 eine Änderung des Magnetfeldes bewirken, so dass die Leseeinrichtung 14 erfassen kann, ob an einer Position eine Ausnehmung 16, 20 vorliegt oder ein Steg 17, 21. So funktioniert das Auslesen der Codierscheibe 18 entsprechend dem Auslesen der Codierhülse 12.

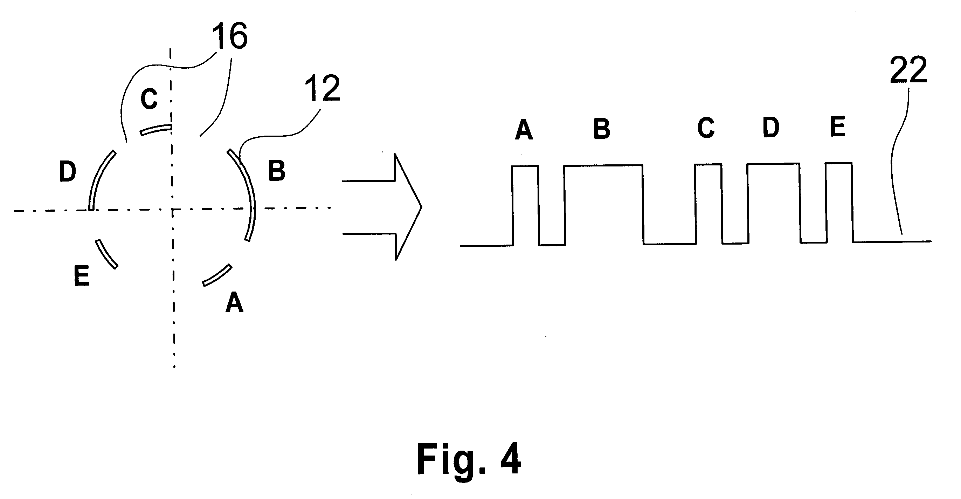

Fig. 4 zeigt eine Schnittansicht durch die Codierhülse 12 gemäß Fig. 2. Zwischen den Ausnehmungen 16 finden sich Stege A bis E der Hülse 12. Diese Stege A bis E weisen wie auch die Ausnehmungen 16 in Umfangsrichtung unterschiedliche Breiten auf, wodurch eine bestimmte Codierung erreicht wird. Bei einer anderen Codierung weisen die Ausnehmungen 16 und die Stege A bis E andere Breiten auf. Je nach Auflösevermögen der Leseeinrichtung 14 können die in Umfangsrichtung erfassbaren Breiten von Ausnehmungen 16 und Stegen A bis E sehr fein gewählt werden, wodurch eine große Anzahl unterschiedlicher Codierungen erreicht werden kann. Wenn die Hülse 12 bei ihrer Rotation die Leseeinrichtung 14 passiert, erfasst die Leseeinrichtung 14 die durch die Ausnehmungen 16 bzw. die dazwischenliegenden Stege A bis E bewirktenMagnetfeldänderungen und gibt einen korrespondierenden Signalverlauf 22 aus, wie er in Fig. 4 rechts dargestellt ist. Dieser Signalverlauf 22 ist ein digitales Signal, welches von einer Steuereinrichtung weiterverarbeitet werden kann.

Figur 5 zeigt eine zweite Ausführungsform einer Codierhülse 23, bei welcher die Codierelemente in Form von Ausnehmungen 24 und Stegen 25 in linearer Richtung in Richtung der Achse R angeordnet sind. Die Ausbildung der Codierung und auch das Auslesen entspricht im Wesentlichen den anhand von Figuren 2 und 3 erläuterten Ausgestaltungen, bei welchen die Codeelemente in Umfangsrichtung bzw. auf einer Kreislinie angeordnet sind. Bei der Ausführungsform gemäß Figur 5 erfolgt das Auslesen des Codes nicht durch Drehung des Werkzeuges relativ zu einem feststehenden Geräteteil wie einem Handgriff, sondern durch axiale Linearbewegung in Richtung X parallel zu der Achse R. Eine solche Bewegung erfolgt beispielsweise beim Einsetzten oder Herausnehmen des Werkzeuges aus einem Handgriff. Diese Ausgestaltung eignet sich auch für feststehende Werkzeuge, welche nicht rotieren sollen. Ferner ist eine Kombination der Codierung gemäß Figur 5 mit einem der anhand der Figuren 2 und 3 erläuterten Beispiele möglich. Bei Kombination mit dem Ausführungsbeispiel gemäß Figur 2 können anstelle der inneren die Stege 25 zusammenhaltenden Wandung , die in Achsrichtung angeordneten Stege 17 gemäß Figur 2 angeordnet sein. Bei dieser Anordnung sind dann die Stege 17 und 25 rechtwinklig gekreuzt zueinander angeordnet. Bei einer Kombination der Ausführungsbeispiele gemäß Figuren 3 und 5 kann beispielsweise die Stirnseite der Scheibe 18, welche der Achse R zugewandt ist, mit Stegen 25 gemäß der Figur 5 versehen sein. Diese Kombinationen der verschiedenen Codierungen ermöglichen zum einen die Ausbildung komplexerer Codes mit größerer Anzahl verschiedener Codierungen, zum anderen ist es möglich verschiedene Codes bei verschiedenen Bewegungsrichtungen des Werkzeuges auszulesen.

Anstatt lediglich Ausnehmungen oder Stege in der Hülse 12 oder der Scheibe 18 vorzusehen, können die einzelnen Codeelemente so ausgebildet sein, dass es mehr als zwei mögliche Zustände des Magnetfeldes gibt. Auf diese Weise kann eine noch größere Informationsmenge in der Codierung untergebracht werden, wobei von der Leseeinrichtung 14 dann ein entsprechend analoges Signal ausgegeben werden kann. Die hier gezeigte einfachste Form der digitalen Codierung erkennt in der Hülse 12 bzw. der Scheibe 18 lediglich zwei Zustände, nämlich "Material" oder "kein Material". Entsprechend kann die Codierung beispielsweise auch optisch ausgebildet werden, indem die zwei Zustände beispielsweise "reflektierend" und "nicht reflektierend" bedeuten, was von einem entsprechenden optischen Sensor erfasst werden kann. Bei allen möglichen und bekannten Ausgestaltungsformen der Codeelemente bleibt jedoch das Grundprinzip der Erfindung gleich, dass die Codeelemente gemeinsam mit einem Werkzeug rotieren und seriell von einem feststehenden Sensor abgetastet bzw. ausgelesen werden.

### Bezugszeichenliste

- 2 -: Welle
- 4 -: Schaft
- 6 -: Werkzeugkopf
- 8 -: Kupplungsteil
- 10 -: Kupplung
- 12 -: Hülse
- 14 -: Leseeinrichtung
- 16 -: Ausnehmung
- 17 -: Steg
- 18 -: Scheibe
- 20 -: Ausnehmung
- 21 -: Steg
- 22 -: Signalverlauf
- 23 -: Hülse
- 24 -: Ausnehmung
- 25 -: Steg

- R -: Rotationsachse
- A bis E -: Stege
- X -: Bewegungsrichtung

## Patentansprüche

1. Codiersystem zur Kennzeichnung von Werkzeugen (2) in medizinischen Geräten, bei welchem an einer Außenseite eines Werkzeuges (2) aufeinanderfolgende Codeelemente (16; 20; 24) angebracht sind und an einem feststehenden Teil eines Gerätes in der Nähe des Werkzeuges eine feststehende Leseeinrichtung (14) angeordnet ist zum Erfassen der aufeinanderfolgenden Codeelemente (16; 20; 24) bei Bewegung des Werkzeuges (2) relativ zu dem feststehenden Teil.

2. Codiersystem nach Anspruch 1, bei welchem das Werkzeug (2) ein rotierendes Werkzeug ist, die Codeelemente (16; 20) in Umfangsrichtung aufeinanderfolgend angeordnet sind und bei Rotation des Werkzeuges (2) relativ zu dem feststehenden Teil von der Leseeinrichtung (14) erfassbar sind.

3. Codiersystem nach Anspruch 1 oder 2, bei welchem die Codeelemente (24) in Längsrichtung des Werkzeuges (2) aufeinanderfolgend angeordnet sind und bei einer Bewegung des Werkzeuges (2) in seiner Längsrichtung relativ zu dem feststehenden Teil von der Leseeinrichtung (14) erfassbar sind.

4. Codiersystem nach einem der vorangehenden Ansprüche, bei welchem die Codeelemente (16; 24) eine Vielzahl zueinander paralleler streifenförmiger Elemente aufweisen, welche in Umfangsrichtung und/oder Längsrichtung des Werkzeuges (2) in unterschiedlichen Abständen zueinander und/oder unterschiedlichen Breiten an der Oberfläche des Werkzeuges (2) zu dessen Kennzeichnung ausgebildet sind.

5. Codiersystem nach einem der vorangehenden Ansprüche, bei welchem die Codeelemente (20) eine Vielzahl von streifenförmigen oder kreissegmentförmigen Elementen (20) aufweisen, welche mit unterschiedlichen Abständen zueinander und/oder unterschiedlichen Breiten ausgebildet sind und entlang einer Kreislinie in einer Ebene quer zur Drehachse (R) des Werkzeuges (2) an dem Werkzeug (2) angeordnet sind.

6. Codiersystem nach einem der vorangehenden Ansprüche, bei welchem die Codeelemente (16; 20; 24) als Stege (17; 21; 25) ausgebildet sind.

7. Codiersystem nach einem der vorangehenden Ansprüche, bei welchem die Codeelemente (16; 20; 24) an einer Hülse (12; 23) oder Scheibe (18) ausgebildet sind, welche mit dem Werkzeug (2) drehfest verbunden ist.

8. Codiersystem nach einem der vorangehenden Ansprüche, bei welchem das Werkzeug einen feststehenden Gehäuseteil (4; 8) und einen darin angeordneten beweglichen Teil (2) aufweist, wobei die Codeelemente (16; 20; 24) im Inneren des Gehäuseteils (4; 8) angeordnet sind.

9. Codiersystem nach Anspruch 8, bei welchem die Codeelemente (16; 20; 24) im Inneren des beweglichen Teils (2) angeordnet sind.

10. Codiersystem nach einem der vorangehenden Ansprüche, bei welchem die Codeelemente (16; 20; 24) magnetfeldbeeinflussend oder optisch erfassbar ausgebildet sind.

11. Verfahren zur Kennzeichnung von Werkzeugen (2) in medizinischen Geräten, bei welchem an einem Werkzeug (2) aufeinanderfolgende Codeelemente (16; 20; 24) angeordnet werden, welche das Werkzeug (2) betreffende Informationen angeben, und zum Auslesen der Informationen das Werkzeug (2) relativ zu einem feststehenden Teil eines Gerätes bewegt wird, wobei die Codeelemente (16; 20; 24) eine feststehend ausgebildete Leseeinrichtung (14) passieren, welche die Codeelemente (16; 20; 24) zum Auslesen der Informationen erfasst.

12. Verfahren nach Anspruch 10, bei welchem die Codeelemente (16; 20) auf einer zur Drehachse (R) des Werkzeuges (2) konzentrischen Kreisbahn angeordnet werden und das Werkzeug (2) zum Auslesen relativ zu dem feststehenden Teil eines Gerätes gedreht wird.

13. Verfahren nach Anspruch 10 oder 11, bei welchem die Codeelemente (24) in Längsrichtung des Werkzeuges (2) aufeinanderfolgend angeordnet werden und bei einer Linearbewegung des Werkzeuges (2) in dessen Längsrichtung relativ zu dem feststehenden Teil des Gerätes von der Leseeinrichtung (14) ausgelesen werden.
